# EUROPEAN PATENT APPLICATION

(11) **EP 2 179 725 A1**
(43) Date of publication of application: **28.04.2010**
(21) Application number: 08167411.1
(22) Date of filing: 23.10.2008
(51) Int. Cl.: A61K 9/14, A61K 9/20, A61K 31/4015

(54) **Pharmaceutical composition comprising levetiracetam**

(71) Applicant: LEK Pharmaceuticals d.d., 1526 Ljubljana (SI)
(72) Inventor: The designation of the inventor has not yet been filed
(74) Representative: Kosak, Alenka

(57) **Abstract**

The present invention relates to a pharmaceutical composition comprising levetiracetam and to a process for its preparation.

## Description

### Field of the Invention

The present invention relates to a pharmaceutical composition comprising levetiracetam and to a process for its preparation.

### Description of the background art

Levetiracetam is disclosed as a protective agent for the treatment and the prevention of hypoxic and ischemic type aggressions of the central nervous system in the European patent No. EP 0 162 036 A and has the following formula.

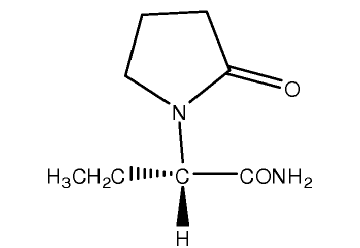

The chemical name of levetiracetam, a single enantiomer, is (-)-(S)-a-ethyl-2-oxo-1-pyrrolidine acetamide, its molecular formula is C₈H₁₄N₂O₂ and its molecular weight is 170.21.
Levetiracetam is marketed under the registered trademark Keppra®. Levetiracetam is available as 250 mg, 500 mg, 750 mg and 1000 mg tablets and as a clear, colorless, grape-flavored liquid (100 mg/ml) for oral administration. KEPPRA tablets typically comprise the labeled amount of levetiracetam, colloidal silicon dioxide, croscarmellose sodium, magnesium stearate, polyethylene glycol 4000, polyvinylpyrrolidone, talc, titanium dioxide, and coloring agents.

In the case of levetiracetam high API loads are desirable, which however are often connected with problems such as bad compressibility, low flowability, sticking and agglomeration of active ingredient, problems being more pronounced with high strength dosage forms (e. g. 750, 1000 mg tablets).

In order to keep the weight of the tablet at a desirable low range such as, for example, at a range of less than about 1000 mg in view of the situation that relatively larger tablets are less preferred in terms of patient compliance, the amount of excipients should be controlled. On the other hand, excipients are normally necessary to achieve satisfactory technological properties. In the case of levetiracetam, especially when used at high API loads, the achievement of a good balance of technological properties, in particular appropriate flowability and compressibility, and product properties, in particular dissolution performance, is challenging.

The object of the present invention is concerned with the problem of meeting a good balance of properties.

### Summary of the invention

The aspects, advantageous features and preferred embodiments of the present invention summarized in the following items, respectively alone or in combination, further contribute to solving the object of the invention:
(1) A pharmaceutical composition, comprising levetiracetam having a particle size d(0.5) of about 120 - 200 µm, and optionally an excipient.
(2) A pharmaceutical composition comprising levetiracetam particles subjected to fluid bed granulation together with excipients.
(3) The pharmaceutical composition according to any one of previous two items wherein the excipient is selected from the group consisting of polyvinylpyrrolidone, microcrystalline cellulose, croscarmellose sodium, crospovidone, colloidal silicon dioxide, magnesium stearate, talc, and mixtures thereof.
(4) The pharmaceutical composition according to any one of previous items, wherein the excipients at least include: polyvinylpyrrolidone, microcrystalline cellulose, colloidal silicon dioxide, magnesium stearate, talc, and a disintegrant selected from croscarmellose sodium and crospovidone, in particular wherein said excipients consist of: polyvinylpyrrolidone, microcrystalline cellulose, colloidal silicon dioxide, magnesium stearate, talc, and croscarmellose sodium.
(5) The pharmaceutical composition according to any one of previous items, wherein the pharmaceutical composition is in the form of a tablet.
(6) A process for the preparation of levetiracetam tablets comprising the steps of:
   a) granulation of levetiracetam having a particle size d(0.5) of about 120 - 200 µm with a binder;
   b) addition of one or more other excipients,
   c) compressing a mixture obtained in step b) into tablet.
(7) The process according to the previous item, wherein the binder contains at least polyvinylpyrrolidone.
(8) The process according to the previous two items, wherein the granulation in step
   a) is a fluid bed granulation.
(9) The process according to any one of previous three items, wherein tablets are subsequently coated with a coating.
(10) A process for the preparation of pharmaceutical composition comprising levetiracetam, preferably in tablet dosage form, comprising fluid bed granulation.
(11) The process according to the previous item, wherein the levetiracetam subjected to fluid bed granulation has a particle size d(0.5) of about 120 - 200 µm
(12) The process according to the previous two items, wherein the pharmaceutical composition is prepared in a tablet dosage form.
(13) A levetiracetam-containing pharmaceutical tablet, characterized by a pH-independent dissolution profile defined by a dissolution variation of levetiracetam of maximally 5%, when tested using USP apparatus 2, placing 4 samples having same levetiracetam tablet formulations in 900 ml of artificial juice at 37 ± 0.5°C with paddle speed of 50 rpm under conditions 0.1 M HCl, pH 4.5, pH 6.8 and water, respectively, and subsequently measuring the respective dissolution percentage after 30 min.
(14) A fabrication system for levetiracetam-containing pharmaceutical tablets, capable of producing levetiracetam tablets having respectively dose-different levetiracetam doses of at least two among doses of 250 mg, 500 mg, 750 mg and 1000 mg, the fabrication system being characterized by providing tablets with a dose-independent dissolution profile defined by a dissolution variation of the aforementioned different levetiracetam tablets of maximally 5%, tested using USP apparatus 2, placing the tablets having respectively different levetiracetam doses in 900 ml of artificial juice at 37 ± 0.5°C with paddle speed of 50 rpm at a pH of 6.8, and subsequently measuring the respective dissolution percentage after 30 min.
(15) The fabrication system according to item (14), which is capable of producing levetiracetam tablets having respectively dose-different levetiracetam doses of all of 250 mg, 500 mg, 750 mg and 1000 mg doses.
(16) The fabrication system according to item (14) or (15), comprising a fluid bed granulation device.

### Description of further Advantages and Preferred Embodiments of the Invention:

In the following, the present invention will be described in more detail by preferred embodiments and examples while referring to the attached drawings, noting, however, that these embodiments, examples and drawings are presented for illustrative purposes only and shall not limit the invention in any way

Fig. 1 shows dissolution profiles of pharmaceutical formulation obtained in Example 8 at different pH (in 0.1 M HCl, at pH 4.5, at pH 6.8 and in water).

Fig. 2 shows dose-independent dissolution profiles of levetiracetam 250, 500, 750, and 1000 mg tablets, prepared according to present invention, at pH 6.8

According to the present invention, it was surprisingly found that selecting the specific size of levetiracetam particles falling in the range of d(0.5) of about 120 - 200 µm markedly influences levetiracetam dissolution in a beneficial manner. Further, this concept is significantly inter-related with processing technology. When choosing such levetiracetam particles for granulation, a good balance of technological properties, notably appropriate flowability and compressibility, and product properties such as dissolution performance can be achieved. Moreover, tablet mass at variable levetiracetam dose levels, including e.g. levetiracetam doses of 250 mg, 500 mg, 750 mg and 1000 mg, can be reproducibly obtained. Further advantages achievable by the pharmaceutical compositions disclosed herein are robustness of product properties, defined by a low intervariability of product properties such as mass, hardness, disintegration time and friability. An alternative contribution to such good balance of properties, optionally observed in combination with the size control for further enhancing the beneficial effects, is when fluid bed granulation is chosen as the granulation technology, distinct from wet granulation, dry granulation or direct compression. Fluid bed granulation, relative to other process technologies, favours to obtain pharmaceutical formulation such as tablet of high porosity and enables high surface area and good disintegration, which constitute relevant factors for the dissolution process of levetiracetam as a *per se* soluble compound, consequently favouring good dissolution performance independent from (but optionally in addition to) the size control. Use of processing technology other than fluid bed granulation, such as wet granulation, can nevertheless be compromised when observing the aforementioned specific size range of levetiracetam particles. The present invention allows a compression into high API load if desired, and compression into tablet cores readily coatable by film coatings. A further advantage common to selecting either the particular API size range or the fluid bed process technology, is that a limited range and limited respective amounts of optional and, if used, preferable excipients can be used for processing, thereby making possible high API load according to desire and demand.

With the pharmaceutical composition according to the preferred embodiment of the invention comprising levetiracetam with a particle size d(0.5) of about 120 - 200 µm, the pharmaceutical composition is robust, shows acceptable dissolution profiles and resistance to chipping.

When too much levetiracetam particles are present whose particle size is lower than about 120 µm, sticking and agglomeration during processing as well as problems with compressibility occur. When too much levetiracetam particles are present whose particle size is larger than about 200 µm, coating of compressed tablets is rendered difficult. Therefore, it is observed that when levetiracetam with a particle size d(0.5) of about 120 - 200 µm is used the above mentioned problems are circumvented. Preferably levetiracetam with a particle size d(0.1) of about 50 - 100 µm, d(0.5) 120 - 200 µm of about and d(0.9) of about 250 - 450 µm is used.
The term d(0.1) refers to the particle size which at least 10% of the particles are within, d(0.5) refers to the particle size which at least 50% of the particles are within, d(0.9) refers to the particle size which at least 90% of the particles are within the aforementioned size ranges, respectively.

The pharmaceutical composition of the present invention includes levetiracetam and at least one pharmaceutically acceptable excipient. Pharmaceutically acceptable excipients include but are not limited to such as binders, disintegrants, fillers, glidants, antiadhesive agents, lubricants, etc.

Various useful binders include but are not limited to hydroxypropylcellulose (Klucel™-LF), hydroxypropyl methylcellulose (Methocel™), polyvinylpyrrolidone (PVP-K25, PVP-K29, PVP-K30), powdered acacia, gelatin, guar gum, carbomer (e.g. carbopol), methylcellulose, polymethacrylates, and starch. Preferred binder is polyvinylpyrrolidone.

Various useful disintegrants include but are not limited to carmellose calcium, carboxymethylstarch sodium, croscarmellose sodium, crospovidone, examples of commercially available crospovidone products including but not limited to crosslinked povidone, Kollidon™ CL, Polyplasdone™ XL, XL-10, and INF-10 and low-substituted hydroxypropylcellulose. Examples of low-substituted hydroxypropylcellulose include but are not limited to low-substituted hydroxypropylcellulose LH11, LH21, LH31, LH22, LH32, LH20, LH30, LH32 and LH33. Preferred disintegrants are croscarmellose sodium and crospovidone.

Various useful fillers include but are not limited to starches, lactose, mannitol, cellulose derivatives and the like. Different grades of lactose include but are not limited to lactose monohydrate and lactose anhydrous. Different grades of starches included but are not limited to maize starch, potato starch, rice starch, wheat starch, pregelatinized starch, fully pregelatinized starch. Different cellulose compounds that can be used include microcrystalline cellulose and powdered cellulose. Examples of microcrystalline cellulose products include but are not limited to Avicel™ PH 101, PH102, PH301, PH302 and PH-F20, microcrystalline cellulose 114, and microcrystalline cellulose 112. Preferred filler is microcrystalline cellulose.

Various useful glidants include but are not limited to starches, colloidal silicon dioxide and talc. Preferred glidant is colloidal silicon dioxide.

Various useful antiadhesive agents include but are not limited to colloidal silicon dioxide and talc. Preferred antiadhesive agent is talc.

Various suitable lubricants include but are not limited to stearic acid, talc and magnesium stearate. Preferred lubricant is magnesium stearate.

The pharmaceutical composition according to a preferred embodiment include as excipients at least: polyvinylpyrrolidone, microcrystalline cellulose, colloidal silicon dioxide, magnesium stearate, talc, and a disintegrant selected from croscarmellose sodium and crospovidone, and according to a further preferred embodiment the excipient consist only of: polyvinylpyrrolidone, microcrystalline cellulose, colloidal silicon dioxide, magnesium stearate, talc, and croscarmellose sodium.

A pharmaceutical compositions according to the present invention is preferably in solid form, including tablets, capsules, caplets, lozenges and sachets. Tablets may be suitably coated (film coated tablets, pills). Capsule formulations may cover both soft and hard capsules. A pharmaceutical compositions according to the present invention is preferably in the form of tablet, preferably coated tablet.

In another embodiment the present invention relates to a process for the preparation of levetiracetam tablets comprising the step of:
a) granulation of levetiracetam having a particle size d(0.5) of about 120 - 200 µm with a binder
b) addition of one or more other excipients,
c) compressing a mixture obtained in step b) into tablets.

In step a) preferably fluid bed granulation is used. Preferred granulation liquid is demineralised water.

Mixture obtained after step a) is dried and optionally sieved.

Preferably in step c) tablets are compressed on rotary compressing machine. The tablets can form cores suitable for further processing.

Tablet cores may be subsequently film coated with appropriate film coating material. Any known method for film coating, known in the field of the pharmaceutical technology, may be used.

A pharmaceutical composition of the present invention comprises preferably 250, 500, 750 or 1000 mg of levetiracetam as the respective dose.

In another embodiment the present invention relates to a tablets that are characterized with a pH independent dissolution profile of levetiracetam, tested using USP apparatus 2, placing the tablets in 900 ml of artificial juice (0.1 M HCl, pH 4.5, pH 6.8 and water) at 37 ± 0.5°C with paddle speed of 50 rpm. The levetiracetam tablets disclosed herein allow a unique pH independent dissolution profile, even realizing maximally 5 % variation among samples after 30 min of the aforementioned four different test conditions.

In another embodiment the present invention relates to tablets that are characterized with dose-independent dissolution profile, tested using USP apparatus 2, placing the tablets in 900 ml of artificial juice (pH 6.8) at 37 ± 0.5°C with paddle speed of 50 rpm. Likewise, the levetiracetam tablets disclosed herein allow a unique dose-independent dissolution profile, even realizing maximally 5 % variation among different samples containing 250, 500, 750 or 1000 mg of levetiracetam respectively after 30 min of the test conditions.

The present invention can thus provide a beneficial fabrication system for levetiracetam-containing pharmaceutical tablets, capable of producing levetiracetam tablets having respectively different levetiracetam doses selected among doses of 250 mg, 500 mg, 750 mg and 1000 mg, wherein the fabrication system can provide such tablets with dose-independent dissolution profile. The fabrication system may be capable of producing respectively dose-different levetiracetam doses of at least two doses, preferably all doses of 250 mg, 500 mg, 750 mg and 1000 mg respectively. The respectively different tablets may contain the same excipients, differing only in the respective levetiracetam doses. The dose differences can be balanced by a corresponding increase of one or more of otherwise used excipient substances. As a contribution to achieve such dose-independent dissolution profile, the fabrication system preferably comprises a fluid bed granulation device, and/or.a device for selecting a desired particle size of levetiracetam particles, which is preferably set to select a d(0.5) particle size range of about 120 - 200 µm. The fabrication system may otherwise include conventional components of tablet fabrication systems.

### Examples

### Examples 1 - 4

Composition of levetiracetam 1000 mg tablets (direct compression)

| Constituent | **Examples** | | | | Function |
|---|---|---|---|---|---|
| | **Example 1** | **Example 2** | **Example 3** | **Example 4** | |
| Levetiracetam | 1000.00 mg | 1000.00 mg | 1000.00 mg | 1000.00 mg | Active |
| Ludipress^{™} | 260.00 mg | / | / | / | Filler, binder |
| Prosolv^{™} (Silicified MCC) | / | 260.00 mg | / | / | Filler, binder |
| Lactose spray dried | / | / | 260.00 mg | / | Filler |
| Avicel PH 102^{™} (MCC) | / | / | / | 260.00 mg | Filler, binder |
| Croscarmellose sodium | 30.00 mg | 30.00 mg | 30.00 mg | 30.00 mg | Disintegrant |
| Colloidal silicon dioxide (Aerosil 200^{™}) | 4.00 mg | 4.00 mg | 4.00 mg | 4.00 mg | Glidant |
| Magnesium stearate | 6.00 mg | 6.00 mg | 6.00 mg | 6.00 mg | Lubricant |

Based on the use of micronized active principle, firstly experiments using direct compression as the manufacturing process were performed. Mixture of active substance and all other excipients, except magnesium stearate, was homogenized and sieved (0.7 mm). Then, magnesium stearate was added and homogeneously mixed and tried to compress into tablets (mass 1300 mg). According to bad compressibility of levetiracetam (20-30%) and high flow density (> 2 ml/g) direct compression with most commonly used excipients in different combinations did not bring acceptable properties of tablets.

### Examples 5 and 6

Composition of levetiracetam 1000 mg tablets (wet granulation)

| Constituent | **Examples** | | Function |
|---|---|---|---|
| | **Example 5** | **Example 6** | |
| **Internals** | | | |
| Levetiracetam | 1000.000 mg | 1000.000 mg | Active |
| PVP K30 (povidone) | 53.320 mg | / | Binder |
| PVP K25 (povidone) | / | 28.000 mg | Binder |
| Crospovidone (Polyplasdon XL^{™}) | / | 19.000 mg | Disintegrant |
| Cellulose microcrystalline (Avicel PH 102^{™}) | 20.000 mg | 19.154 mg | Filler, binder |
| Purified water | 80.690 mg | 70.000 mg | Granulation fluid |
| **Externals** | | | |
| Cellulose microcrystalline (Avicel PH 102^{™}) | 41.834 mg | 29.500 mg | Filler |
| Crospovidone (Polyplasdon XL^{™}) | 24.000 mg | 9.500 mg | Disintegrant |
| Talc | 11.893 mg | 11.893 mg | Antiadhesive agent |
| Aerosil 200^{™} (silica, colloidal anhydrous) | 2.973 mg | 2.973 mg | Glidant |
| Magnesium stearate | 5.980 mg | 5.980 mg | Lubricant |
| **Total weight** | **1160.000 mg** | **1126.000 mg** | **Matrix** |

Further experiments were done by wet granulation process
PVP was dissolved in water and added with spraying to the well blended mixture of levetiracetam and avicel. After drying (< 1%) and sieving of granulate throughout sieve 1.0 mm. Afterwards, external excipients were screened through a 0.5 mm screen, added to granulate, and mixed for 3 minutes. Finally, Mg-stearate (0.3 mm) was added and blended. The resulting final blend was compressed into tablets using a rotary tablet press in a controlled environment. Tablets were compressed at a compression weight of 1160 or 1126 mg using capsule-shaped, biconvex punches.

Wet granulation with high amount of API (86.2%) resulted in tablets with acceptable properties but with very low dissolution profiles and long disintegration times (> 15 min).

### Examples 7 and 8:

Composition of Levetiracetam 1000 mg tablets (fluid bed granulation)

| **Internals** | **Example 7** | **Example 8** | Function |
|---|---|---|---|
| Levetiracetam | 1000.000 mg | 1000.000 mg | Active |
| PVP K30 (povidone) | 53.320 mg | 53.000 mg | Binder |
| Purified water | 485.000 mg | 500.000 mg | Granulation fluid |
| **Externals** | | | |
| Cellulose microcrystalline (Avicel PH 102^{™}) | 61.834 mg | 65.000 mg | Filler |
| Croscarmellose sodium (Ac-di-Sol^{™}) | / | 22.000 mg | Disintegrant |
| Crospovidone (Polyplasdon XL^{™}) | 24.000 mg | / | Disintegrant |
| Talc | 11.893 mg | 11.000 mg | Antiadhesive agent |
| Aerosil 200^{™} (silica, colloidal anhydrous) | 2.973 mg | 3.000 mg | Glidant |
| Magnesium stearate | 5.980 mg | 6.000 mg | Lubricant |
| **Weight** | **1160.000 mg** | **1160.000 mg** | Matrix |
| Film coating | 40.000 mg | 40.000 mg | |
| **Total weight** | **1200.000 mg** | **1200.000 mg** | |

For further experiments crospovidone and croscarmellose sodium as disintegrants were used, and fluid bed granulation as manufacturing process.
Levetiracetam was sieved (0.71 mm) and put in fluid bed granulator. Solution of water with polyvinylpyrrolidone was added throughout spray nozzle (fluid bed granulation). During the process, solution was sprayed with 10 - 13 g/min, incoming air temperature was 50 - 55ºC, product temperature was 30 - 35ºC, and outgoing temperature was 25 - 30ºC, using 1.0 mm upper nozzle system. Speed of incoming air was 1.0 - 3.0 m/s, and interval of shaking was 3 s every 30 s. After ending spraying water/PVP, mass was dried below 0.5% of moisture in fluid bed granulator and material was subsequently sieved (1.00 mm). Then, avicel, crospovidone or croscarmellose sodium, talc, and aerosil (0.5 mm) were added to granulated part, and mixed for 5 minutes at 14 rpm. Magnesium stearate (0.3 mm) was added and blended (1 min, 14 rpm) at the end. The resulting final blend was compressed into tablets using a rotary tablet press in a controlled environment.
Tablets were subsequently film coated.
Tablets of the present invention prepared according to Example 8 showed pH independent dissolution profile.

Particle size distribution of levetiracetem was characterized by laser diffraction (Malvern Mastersizer S). Sample cell was small volume sample dispersion cell MS1, presentation was 3NHE, solvent was hexane, stirrer speed was 2000 rpm. The following procedure was used:
Fill the sample cell with hexane, add 100 mg of sodium bis (2-ethylhexyl) sulfosuccinate, align the sizer and measure the background. Add sample into the sample cell until proper obscuration is achieved (10-30%). Analyze when the signal from detectors is stable.

### Dissolution tests:

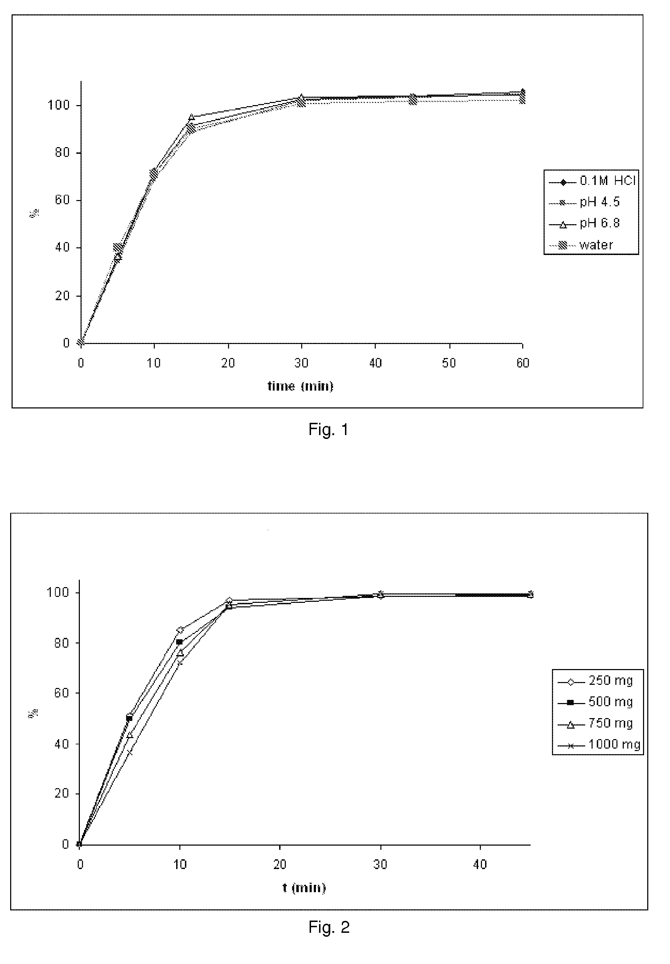

Tablets of the present invention were subjected to dissolution testing with attention to variability of dissolution at different pH (in 0.1M HCl, at pH 4.5, at pH 6.8 and in water) and to variability of dissolution at different strengths (250, 500, 750, and 1000 mg tablets at pH 6.8).
The results are shown in Figures 1 and 2.
Dissolution properties were evaluated by *in vivo* relevant dissolution test. USP apparatus 2 was used for testing. 900 ml of artificial juice (0.1 M HCl, pH 4.5, pH 6.8 and water) is placed in the dissolution vessel, mixed with a paddle at 50 rpm and kept at 37 ± 0.5°C. Samples were taken from the dissolution vessel at regular time intervals and the concentrations of levetiracetam were analyzed by HPLC.

## Claims

1. A pharmaceutical composition, comprising levetiracetam having a particle size d(0.5) of about 120 - 200 µm and optionally an excipient.

2. A pharmaceutical composition comprising levetiracetam particles subjected to fluid bed granulation together with excipients.

3. A pharmaceutical composition according to any one of claims 1 and 2, wherein the excipient is selected from the group consisting of polyvinylpyrrolidone, microcrystalline cellulose, croscarmellose sodium, crospovidone, colloidal silicon dioxide, magnesium stearate, talc, and mixtures thereof.

4. A pharmaceutical composition according to any one of claims 1 to 3, wherein a pharmaceutical composition is in the form of a tablet.

5. A process for the preparation of levetiracetam tablets comprising the steps of:
a) granulation of levetiracetam with a particle size d(0.5) of about 120 - 200 µm with a binder;
b) addition of one or more other excipients,
c) compressing a mixture obtained in step b) into tablets.

6. A process according to claim 5, wherein the granulation in step a) is wet granulation, preferably fluid bed granulation.

7. A process according to any of claims 5 and 6, wherein the tablets are subsequently coated with a coating.

8. A process for the preparation of pharmaceutical composition comprising levetiracetam, the process comprising fluid bed granulation.

9. The process according to claim 8, wherein the levetiracetam subjected to fluid bed granulation has a particle size d(0.5) of about 120 - 200 µm

10. The process according to claim 8 or 9, wherein the pharmaceutical composition is prepared in a tablet dosage form.

11. A levetiracetam-containing pharmaceutical tablet, **characterized by** a pH-independent dissolution profile defined by a dissolution variation of levetiracetam of maximally 5%, when tested using USP apparatus 2, placing 4 samples having same levetiracetam tablet formulations in 900 ml of artificial juice at 37 ± 0.5°C with paddle speed of 50 rpm under conditions 0.1 M HCl, pH 4.5, pH 6.8 and water, respectively, and subsequently measuring the respective dissolution percentage after 30 min.

12. A fabrication system for levetiracetam-containing pharmaceutical tablets, capable of producing levetiracetam tablets having respectively dose-different levetiracetam doses of at least two among doses of 250 mg, 500 mg, 750 mg and 1000 mg, the fabrication system being **characterized by** providing tablets with a dose-independent dissolution profile defined by a dissolution variation of the aforementioned different levetiracetam tablets of maximally 5%, tested using USP apparatus 2, placing the tablets having respectively different levetiracetam doses in 900 ml of artificial juice at 37 ± 0.5°C with paddle speed of 50 rpm at a pH of 6.8, and subsequently measuring the respective dissolution percentage after 30 min.

13. The fabrication system according to claim 12, comprising a fluid bed granulation device.
